# EUROPEAN PATENT SPECIFICATION

(11) **EP 3 424 474 B1**
(45) Date of publication and mention of the grant of the patent: **21.04.2021**
(21) Application number: 16892526.1
(22) Date of filing: 01.03.2016
(51) Int. Cl.: A61F 13/15, A61F 13/49, A61F 13/496

(54) **METHOD FOR PRODUCING ABSORBENT ARTICLE**
VERFAHREN ZUR HERSTELLUNG EINES SAUGFÄHIGEN ARTIKELS
PROCÉDÉ DE PRODUCTION D'UN ARTICLE ABSORBANT

(43) Date of publication of application: 09.01.2019
(73) Proprietor: Unicharm Corporation, Shikokuchuo-shi, Ehime 799-0111 (JP)
(72) Inventor: KAWAZU, Fumihito, Kanonji-shi Kagawa 769-1602 (JP); NINOMIYA, Akihide, Kanonji-shi Kagawa 769-1602 (JP); SHIBATA, Kenji, Kanonji-shi Kagawa 769-1602 (JP)
(74) Representative: Staeger & Sperling Partnerschaftsgesellschaft mbB
(86) International application number: PCT/JP2016/056315
(87) International publication number: WO 2017/149682

(56) References cited:
- WO-A1-2004/031053
- JP-A- S4 840 522
- JP-A- H09 110 019
- JP-A- 2003 250 826
- JP-A- 2009 061 046
- JP-A- 2015 532 183
- JP-A- 2015 532 184
- JP-U- H04 828
- US-A1- 2003 062 113
- US-B2- 6 723 035

## Description

### Technical Field

The present invention relates to a method for manufacturing an absorbent article.

### Background Art

Pants-style disposable diapers are known that include an absorbent main body to absorb excrement, and front and back waist sections that are arranged around the waist of the wearer when the diapers are being worn. When such pull-on disposable diapers are distributed in the marketplace, normally portions of the front and back waist sections that project out to the lateral direction outside of the absorbent main body (also referred to as side panels or side flaps) are folded in to achieve a compact shape before packaging. For example, Patent Document 1 discloses technology in which a side panel folding device or the like is used on pull-on diapers being conveyed along a conveying direction in a manufacturing process to push the side panels inward from the outside toward the inside in the lateral direction.

### Citation List

### Patent Literature

Patent Literature 1: Japanese Unexamined Patent Application Publication 2013-523326. Further prior art in this technical field is disclosed in documents US 6,723,035 B2 and US 2003/0062113 A1.

### Summary of Invention

### Technical Problem

However, in the method of Patent Document 1, the side panels (side flaps) that were pushed inward sometimes return to their original positions when the inward pushing by the side panel folding device ceases. Namely, the side panels that have been folded inwards from the outside in the lateral direction sometimes do not maintain a folded-in state, and return to sticking out in the lateral direction. It is difficult in such circumstances to fold the pull-on diapers compactly.

In consideration of the above issues, an object of the invention is to compactly fold pull-on diapers equipped with side flaps.

### Solution to Problem

In order to achieve the above object, a main invention is defined in claim 1 and refers to a method for manufacturing an absorbent article for manufacturing a pull-on absorbent article having a height direction, a lateral direction, and a front-back direction intersecting with each other. The absorbent article includes an absorbent main body configured to absorb excrement, a front exterior member arranged at one end side of the absorbent main body, and a back exterior member arranged at another end side of the absorbent main body, with the front exterior member and the back exterior member joined together at a pair of join portions- The method for manufacturing an absorbent article includes: in a state in which the front-back direction of the pull-on absorbent article is aligned in an up-down direction and the pants style absorbent article is being conveyed in a conveying direction along the height direction, pulling at least a portion on the front side of the absorbent main body toward one side in the up-down direction and pulling at least a portion on the back side of the absorbent main body toward the other side in the up-down direction, and folding in the front exterior member and the back exterior member between the front side and the back side of the absorbent main body in the up-down direction until an end portion in the lateral direction at the front side of the absorbent main body is curved toward the other side in the up-down direction and an end portion in the lateral direction at the back side of the absorbent main body is curved toward the one side in the up-down direction.

Other features of the invention will become apparent from the present specification and accompanying drawings.

### Advantageous Effects of Invention

According to the present invention, pull-on diapers with side flaps can be folded compactly-

### Brief Description of Drawings

Fig. 1 is a schematic perspective diagram of a diaper 1 as seen from a front side.
Fig. 2 is a plan view of the diaper 1 in an unfolded and extended state.
Fig. 3 is a schematic cross-section taken along line A-A of Fig. 1.
Fig. 4 is a schematic cross-section taken along line B-B of Fig. 1.
Fig. 5 is a diagram illustrating a manufacturing flow of the diaper 1.
Fig. 6 is a schematic diagram to explain each of the processes when manufacturing the diaper 1.
Fig. 7 is a cross-sectional view to explain a folding-in action performed on side flaps 70.
Fig. 8 is a schematic side view illustrating an example of a configuration of a side flap fold-in mechanism 100.
Fig. 9A and Fig. 9B are diagrams to explain an example of a configuration of a conveying means 110.
Fig. 10A and Fig. 10B are diagrams to explain an example of a configuration of a fold-in means 130.
Fig. 11 is a diagram illustrating an enlargement of region X in Fig. 10B.
Fig. 12A is a schematic plan view of the diaper 1 in a state in which the side flaps 70 have been folded in, as viewed from the front side.
Fig. 12B is a schematic plan view of the diaper 1 in a state in which the side flaps 70 have been folded in, as viewed from the back side.
Fig. 13A and Fig. 13B are diagrams illustrating a modified example of the fold-in means 130.

### Description of Embodiments

At least the following items are apparent from the present specification and accompanying drawings.

A method for manufacturing an absorbent article for manufacturing a pull-on absorbent article having a height direction, a lateral direction, and a front-back direction intersecting with each other. The absorbent article includes an absorbent main body configured to absorb excrement, a front exterior member arranged at one end side of the absorbent main body, and a back exterior member arranged at another end side of the absorbent main body, with the front exterior member and the back exterior member joined together at a pair of join portions. The method for manufacturing an absorbent article includes: in a state in which the front-back direction of the pull-on absorbent article is aligned in an up-down direction and the pants style absorbent article is being conveyed in a conveying direction along the height direction, pulling at least a portion on the front side of the absorbent main body toward one side in the up-down direction and pulling at least a portion on the back side of the absorbent main body toward the other side in the up-down direction; and folding in the front exterior member and the back exterior member between the front side and the back side of the absorbent main body in the up-down direction until an end portion in the lateral direction at the front side of the absorbent main body is curved toward the other side in the up-down direction and an end portion in the lateral direction at the back side of the absorbent main body is curved toward the one side in the up-down direction.

According to such a method for manufacturing an absorbent article, the side flaps configured by the front exterior member and the back exterior member can be folded in until they are positioned deeply inward in the lateral direction (i.e. between the absorbent main body in the front-back direction) to the extent that the lateral direction end portions of the absorbent main body are curved. Thus, the lateral direction size of the absorbent main body is sufficiently small when the side flaps are in a folded-in state, making it easier to package the absorbent article compactly.

In this method for manufacturing an absorbent article, when the front exterior member and the back exterior member are folded in, preferably a portion of overlap in the up-down direction is made where the front exterior member and the back exterior member arranged at one side in the lateral direction overlap with the front exterior member and the back exterior member arranged at another side in the lateral direction.

According to such a method for manufacturing an absorbent article, due to a region of overlap in the up-down direction (front-back direction) arising when the front exterior member and the back exterior member (side flaps) have been folded in, frictional force is readily generated at the overlap region, and the side flaps overlapped in the up-down direction are not liable to become misaligned. This makes it easier to maintain a folded-in state of the side flaps, and makes it easier to fold the absorbent article compactly.

In this method for manufacturing an absorbent article, at the overlapping portion in the up-down direction, where the front exterior member and the back exterior member arranged at the one lateral direction side overlap with the front exterior member and the back exterior member arranged at the other lateral direction side, the front exterior member and the back exterior member are preferably folded inward such that the front exterior member and the back exterior member arranged at the one lateral direction side contact the front exterior member and the back exterior member arranged at the other lateral direction side.

According to such a method for manufacturing an absorbent article, by causing the folded-in side flaps to contact each other in overlap region where the side flaps overlap in the up-down direction (front-back direction), a larger frictional force is readily generated, making it easy to maintain a stronger folded-in state of the side flaps. This makes it easier to fold the absorbent article compactly.

In this method for manufacturing an absorbent article according to the invention, a surface area of a folded-in portion of the front exterior member and the back exterior member folded in between the front side and the back side of the absorbent main body in the up-down direction at a waist opening side in the height direction is larger than a surface area of a folded-in portion of the front exterior member and the back exterior member folded in between the front side and the back side of the absorbent main body in the up-down direction at a crotch side in the height direction.

According to such a method for manufacturing an absorbent article, due to the folded-in amount of the side flaps at the waist opening side of the absorbent article being larger than the folded-in amount of the side flaps at the crotch side thereof, the side flaps are readily overlapped in the up-down direction (front-back direction) at the waist opening side when the side flaps are folded in. This makes it easier to maintain a folded-in state at the waist opening side, and makes it easier to suppress the waist opening of the absorbent article from opening.

In this method for manufacturing an absorbent article, the absorbent main body preferably contracts in the lateral direction due to an elastic region which is stretchable in the lateral direction, and an amount of contraction in the lateral direction of the absorbent main body at the back side is preferably different to an amount of contraction in the lateral direction of the absorbent main body at the front side.

According to such a method for manufacturing an absorbent article, the fold-in position of the side flaps in the lateral direction is made to be different at the back side to at the front side when the side flaps are folded in along the profile of the lateral direction edges of the absorbent main body. This enables the external appearance profile of the absorbent article to be made different at the front side to at the back side, and enables the front and back of the absorbent article to be easily discerned.

In this method for manufacturing an absorbent article, preferably a surface area of a portion where the absorbent main body is pulled toward the one side in the up-down direction at the front side is different to a surface area of a portion where the absorbent main body is pulled toward the other side in the up-down direction at the back side.

According to such a method for manufacturing an absorbent article, the front side and the back side of the absorbent main body can be pulled over respective optimal ranges to match the widths of the absorbent main body contracted in the lateral direction. Positional misalignment of the absorbent article is accordingly not liable to occur, and a stable pulling action can be performed.

In this method for manufacturing an absorbent article, preferably the front exterior member and the back exterior member are folded in between the front side and the back side of the absorbent main body in the up-down direction by a pair of impellers respectively provided at each side of the pull-on absorbent article in a direction intersecting the conveying direction, and preferably the pair of impellers include a portion where the impellers overlap with each other in the up-down direction.

According to such a method for manufacturing an absorbent article, the side flaps are pushed in mechanically by the impellers, and so this makes it easier to maintain a constant folded-in amount of the side flaps. When doing so, the side flaps are folded inward deeply until the left and right impellers overlap with each other in the up-down direction. This enables a region where the side flaps overlap with each other in the up-down direction to be formed with certainty, and makes it easier to maintain a folded-in state of the side flaps.

In this method for manufacturing an absorbent article, preferably the front exterior member and the back exterior member are folded in between the front side and the back side of the absorbent main body in the up-down direction by the pair of impellers pushing against positions on the front exterior member and the back exterior member different from the positions of the join portions.

According to such a method for manufacturing an absorbent article, the join portion having a certain thickness and the fold lines of the side flaps are suppressed from overlapping with each other, thereby reducing the possibility of the folded-in portions of the side flaps becoming thicker or the fold lines becoming difficult to form. This makes it easier to maintain a folded-in state of the side flaps in the absorbent article.

In this method for manufacturing an absorbent article, preferably a rotation shaft of at least one impeller out of the pair of impellers is arranged at a predetermined angle of inclination to the up-down direction.

According to such a method for manufacturing an absorbent article, a gap in the up-down direction between the impellers in the region where the pair of impellers overlaps in the up-down direction is formed with a narrower portion and a wider portion. Smooth action of the impellers is facilitated at the portion where the gap is wider. However, frictional force is readily generated by compression of the side flaps at the portion where the gap is narrower in the up-down direction, which makes it easier to maintain a folded-in state of the side flaps.

In this method for manufacturing an absorbent article, preferably the front exterior member and the back exterior member are folded in between the front side and the back side of the absorbent main body in the up-down direction by ejecting air from a pair of blow nozzles respectively provided at each side of the pull-on absorbent article in a direction intersecting the conveying direction so as to blow the air against the front exterior member and the back exterior member.

According to such a method for manufacturing an absorbent article, due to not requiring impellers to fold in the side flaps, damage or the like to the side flaps due to being pushed in by the impellers is suppressed from occurring.

This method for manufacturing an absorbent article preferably includes: a process in which a front band-shaped member configured by a plurality of the front exterior members connected together contiguously along the lateral direction, and a back band-shaped member configured by a plurality of the back exterior members connected together contiguously along the lateral direction, are conveyed along the lateral direction, and while being conveyed along the lateral direction the front band-shaped member and the back band-shaped member are cut at respective end portions in the lateral direction of the front exterior members and the back exterior members; and a process in which a direction in which the front band-shaped member and the back band-shaped member are being conveyed is turned in direction by a predetermined angle so that when they have been cut the cut front band-shaped members and the cut back band-shaped members are conveyed along the height direction.

According to such a method for manufacturing an absorbent article, the process to manufacture the absorbent article and the process to fold in the side flaps can be executed with good efficiency on the same manufacturing line, enabling manufacture of absorbent articles in a folded state to be performed continuously.

### ===Embodiments===

### <Basic Configuration of Diaper 1>

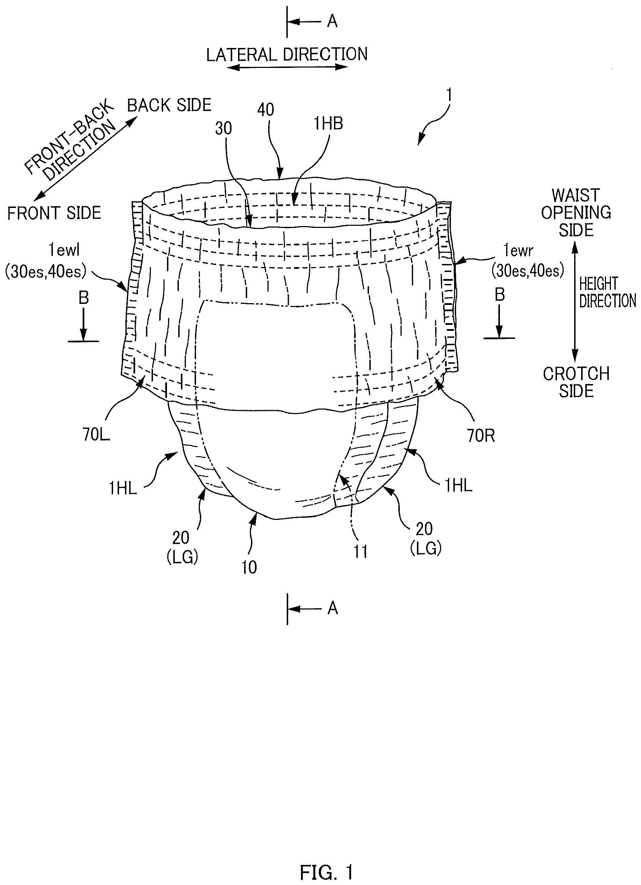

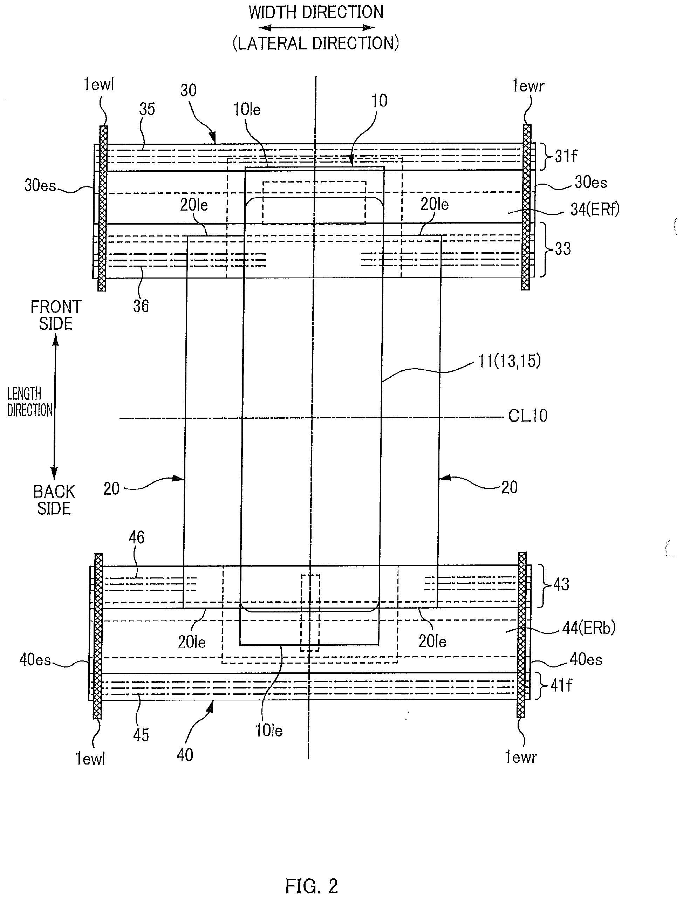

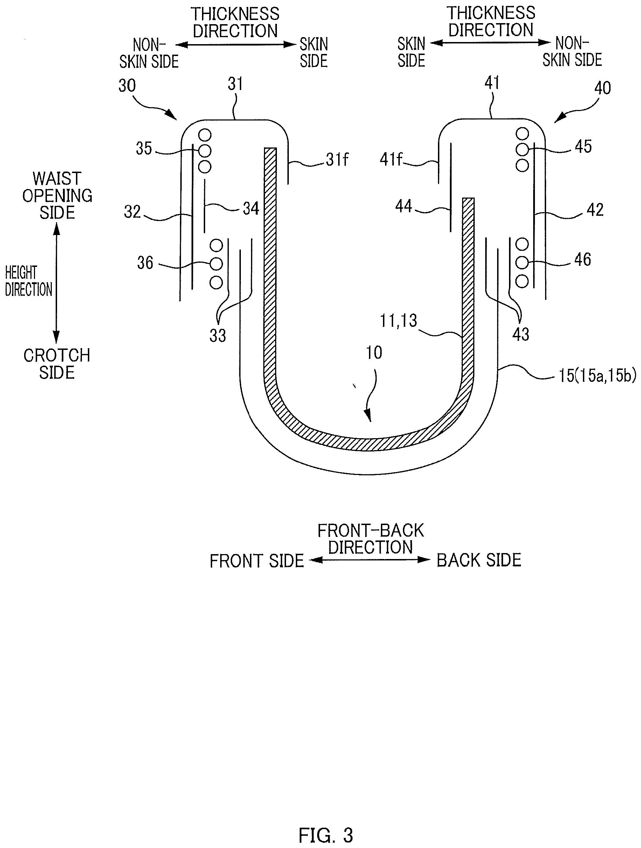

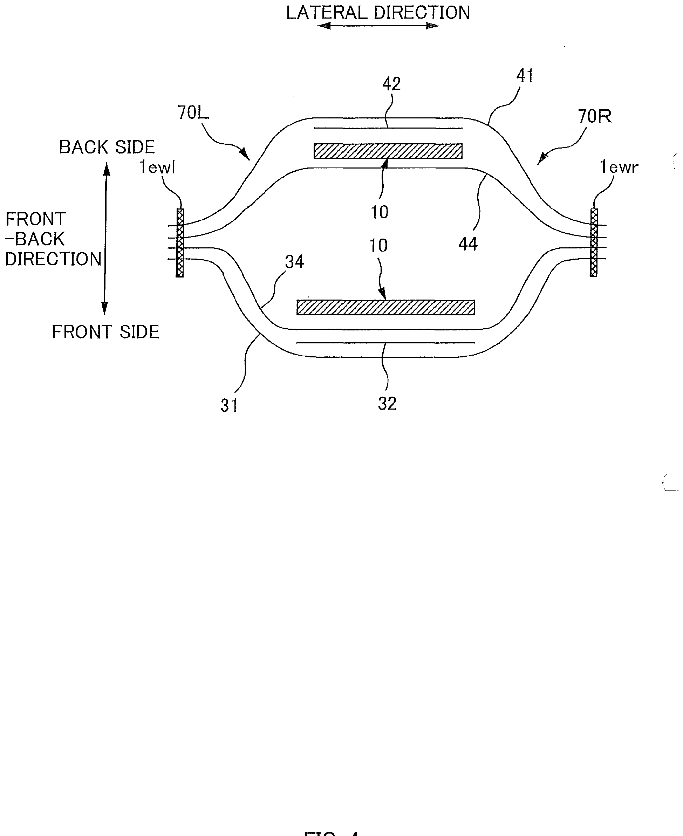

A description will first be given of a basic configuration of a pull-on disposable diaper 1 (also referred to below as "diaper 1"), serving as an example of an absorbent article handled by the present embodiment. Fig. 1 is a schematic perspective diagram of the diaper 1 as seen from a front side. Fig. 2 is a plan view of the diaper 1 in an unfolded and extended state. Fig. 3 is a schematic cross-section taken along line A-A of Fig. 1. Fig. 4 is a schematic cross-section taken along line B-B of Fig. 1. Note that the "unfolded and extended state" in Fig. 2 is a state in which a product (the diaper 1) has been extended so that there are no creases . Specifically, this could be described as a state extended such that the dimensions of each member configuring the diaper 1 have the same length as the dimensions of each of the individual parts themselves, or lengths that are close to such dimensions.

In the pull-on state of Fig. 1, the diaper 1 has three mutually orthogonal directions, a height direction, a lateral direction, and a front-back direction. In the following, one side and another side in the height direction of this pull-on state are referred to as the "waist opening side" and the "crotch side", and the front side and the back side in the front-back direction are referred to as the "front side" and the "back side".

In the unfolded state in Fig. 2, the diaper 1 has a length direction and a width direction of the three mutually orthogonal directions. In the following, one side and another side in the length direction of this unfolded state are also respectively referred to as the "front side" and the "back side". Note that the width direction in the unfolded state as referred to above is the same direction as the lateral direction referred to above with reference to the pull-on state. In the following the width direction is accordingly also sometimes referred to as the "lateral direction". Moreover, the length direction in the unfolded state is a direction along the height direction in the pull-on state. As illustrated in Fig. 3, a direction orthogonal to both the height direction (length direction) and the lateral direction (width direction) is referred to as the "thickness direction", and the side that contacts the skin of the wearer is referred to as the "skin side", and the opposite side thereto is referred to as the "non-skin side".

The diaper 1 of the present embodiment includes an absorbent main body 10, a leg-band member 20, a front exterior member 30, and a back exterior member 40. From the unfolded state of Fig. 2, the absorbent main body 10 is folded in two along a fold position at a predetermined position CL10 in the length direction (height direction) of the absorbent main body 10. The front exterior member 30 and the back exterior member 40 that face each other in this folded-in-two state are joined together by welding or the like at front side edges 30es and back side edges 40es, so as to form a right join portion lewr and a left join portion 1ewl. The front exterior member 30 and the back exterior member 40 are thereby joined together into a ring shape so as to form a pull-on state of the diaper 1 as illustrated in Fig. 1, with a waist opening 1HB and a pair of leg openings 1HL, 1LH.

The absorbent main body 10 has a function to absorb excrement such as urine. As illustrated in Fig. 2, the absorbent main body 10 has a substantially rectangular profile in plan view, and is arranged at the lateral direction center of the diaper 1, with the length direction of the absorbent main body 10 spanning along the height direction of the diaper 1. The absorbent main body 10 includes an absorbent core 11 with liquid absorption properties, a front-face sheet 13 that covers the absorbent core 11 from the skin side, and a back-face sheet 15 that covers the absorbent core 11 from the non-skin side and configures the exterior of the absorbent main body 10. The leg-band members 20 are also provided at each lateral direction side of the absorbent main body 10.

The absorbent core 11 is a member formed by layering a material with liquid absorption properties, and fibers with liquid absorption properties such as pulp fibers can, for example, be used therefor. Note that the absorbent core 11 may, for example, contain a highly absorbent polymer in liquid-absorbent granule form, and may include other materials with liquid absorption properties. The absorbent core 11 may be covered by a liquid permeable sheet such as tissue paper (not illustrated in the drawings).

The front-face sheet 13 is, for example, a liquid permeable nonwoven fabric having a plan view size larger than that of the absorbent core 11. The back-face sheet 15 is also a sheet having a plan view size larger than that of the absorbent core 11. An example thereof is a sheet having a double layer structure in which a leak prevention sheet 15a of a liquid impermeable material such as polyethylene or polypropylene is laminated to an exterior sheet 15b of a nonwoven fabric or the like. Moreover, what is referred to as a barrier cuff LSG (not illustrated in the drawings) may be formed to the absorbent main body 10 of the diaper 1. A barrier cuff LSG is a leak prevention wall erected at both lateral direction end portions of the absorbent main body 10.

The leg-band members 20 function as leg gathers LG, and are band-shaped sheet members arranged so as to run along the height direction at the two lateral direction end portions of the absorbent main body 10. The leg-band members 20 form a portion of the leg openings 1HL of the diaper 1. The leg-band members 20 are, for example, formed by folding a rectangular shaped nonwoven fabric along the height direction. Plural leg-band elastic members (not illustrated in the drawings) , such as rubber threads, are joined between the folded over nonwoven fabric while in a state extended along the height direction. Elasticity is imparted to the leg-band members 20 by the leg-band elastic members, improving the fitabilty of the leg openings 1HL. The length of the leg-band members 20 in the height direction (length direction) is shorter than the length of the absorbent main body 10 in the height direction (length direction) . Namely, the positions of end portions 201e at the two height direction ends of the leg-band members 20 are at the height direction inside of positions at end portions 101e at the two height direction sides of the absorbent main body 10.

The front exterior member 30 is joined to one end side of the absorbent main body 10 in the length direction (height direction) , and is a member that configures a waist section at the front side of the diaper 1. The front exterior member 30 of the present embodiment includes an exterior back sheet 31, a film sheet 32, cover sheets 33, a stretchable sheet 34, waist-gather elastic members 35, and fit-gather elastic members 36.

The exterior back sheet 31 is a sheet member made from a soft sheet material such as a nonwoven fabric and has a substantially rectangular shape in plan view. The height direction upper end portion of the exterior back sheet 31 is formed into a folded-over section 31f (see Fig. 3) by folding the exterior back sheet 31 from the height direction outside toward the height direction inside so as to be folded over in the thickness direction from the non-skin side toward the skin side (from the outside toward the inside in the front-back direction). The film sheet 32 is a sheet member made from a resin and printed with text, designs, and the like, and is arranged so as to be interposed in the thickness direction between the absorbent main body 10 and the exterior back sheet 31 (see Fig. 3 and Fig. 4) . The cover sheets 33 are band-shaped sheet members formed from a nonwoven fabric or the like, and are arranged along the lateral direction at a height direction lower end region of the exterior back sheet 31. The stretchable sheet 34 is a sheet member capable of exhibiting elasticity along the lateral direction, and is formed, for example, from a stretchable nonwoven fabric. The stretchable sheet 34 is arranged at a height direction central portion of the front exterior member 30 in a state extended along the lateral direction. The stretchable sheet 34 forms a front elastic region ERf that imparts elasticity in the lateral direction to the front exterior member 30.

The waist-gather elastic members 35 are elastic members such as rubber threads. Plural of the waist-gather elastic members 35 are joined to a height direction upper end portion of the front exterior member 30 in a state extended along the lateral direction and arranged between the exterior back sheet 31 and the folded-over section 31f. Elasticity is imparted to the waist opening 1HB of the diaper 1 by the elasticity of these waist-gather elastic members 35. The fit-gather elastic members 36 are elastic members such as rubber threads. Plural of the fit-gather elastic members 36 are joined to a height direction lower end portion of the front exterior member 30 in a state extended along the lateral direction and arranged between the exterior back sheet 31 and the cover sheets 33. Elasticity is imparted to a portion of the leg openings 1HL of the diaper 1 by the elasticity of the fit-gather elastic members 36. Note that, as illustrated in Fig. 2, some of the fit-gather elastic members 36 are cut or the like in a region where the fit-gather elastic members 36 overlap with the absorbent main body 10 in the thickness direction, so that this region does not exhibit elasticity. This suppresses the absorbent main body 10 from being excessively contracted in the lateral direction.

The back exterior member 40 is a member joined to the other length direction (height direction) end side of the absorbent main body 10, and configures a waistband section at the back side of the diaper 1. The back exterior member 40 of the present embodiment includes an exterior back sheet 41, a film sheet 42, cover sheets 43, a stretchable sheet 44, waist-gather elastic members 45, and fit-gather elastic members 46. The function and configuration of each member of the back exterior member 40 are substantially the same as those of the front exterior member 30 as described above (see Fig. 3 and Fig. 4), and so detailed description will be omitted thereof.

In the diaper 1, the proportional extension of the stretchable sheet 44 of the back exterior member 40 is set so as to be higher than the proportional extension of the stretchable sheet 34 of the front exterior member 30. Namely, in the diaper 1, the elasticity of a back elastic region ERb formed by the stretchable sheet 44 at the back side is greater than the elasticity of a front elastic region ERf formed by the stretchable sheet 34 at the front side. Thus results in the amount of contraction in the lateral direction of the absorbent main body 10 (the absorbent core 11) being different at the back side and the front side of the diaper 1. Specifically, the absorbent main body 10 is readily contracted in the lateral direction at the back side, and, as illustrated in Fig. 4, the width of the absorbent main body 10 in the lateral direction is wider on the front side. Furthermore, the size and shape of creases that develop on the front exterior member 30 and the back exterior member 40 on the back side are different from that on the front side.

Note that "proportional extension" indicates a degree of extension when the natural length of the elastic member (rubber thread/stretchable sheet) is taken as 1. For example, a proportional extension of 1.2 means that the elastic member is extended from its natural length by an amount of 0.2 times the natural length.

Moreover, in the following description, the portions of the front exterior member 30 and the back exterior member 40 of the diaper 1 that stick out to the outside in the lateral direction from the two lateral direction end portions of the absorbent main body 10 are referred to as a pair of side flaps 70. In Fig. 1, the diaper 1 includes a right side flap 70R sticking out to the right side, and a left side flap 70L sticking out to the left side.

### <Method for Manufacturing the Diaper 1>

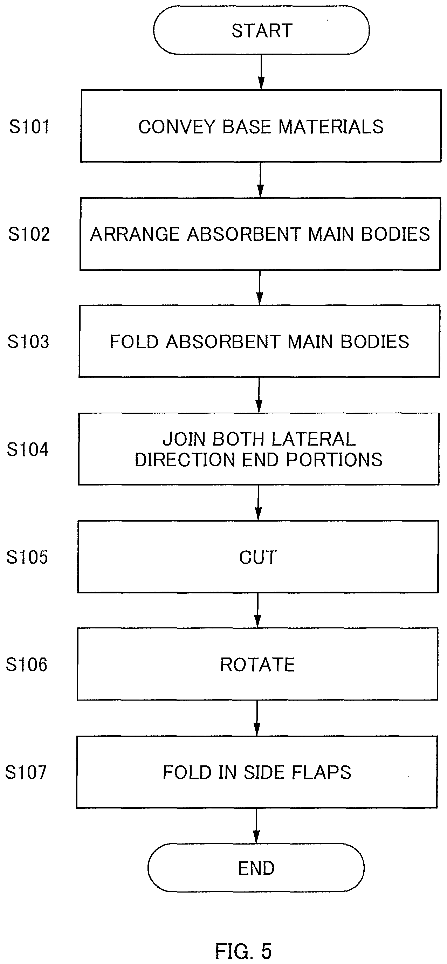

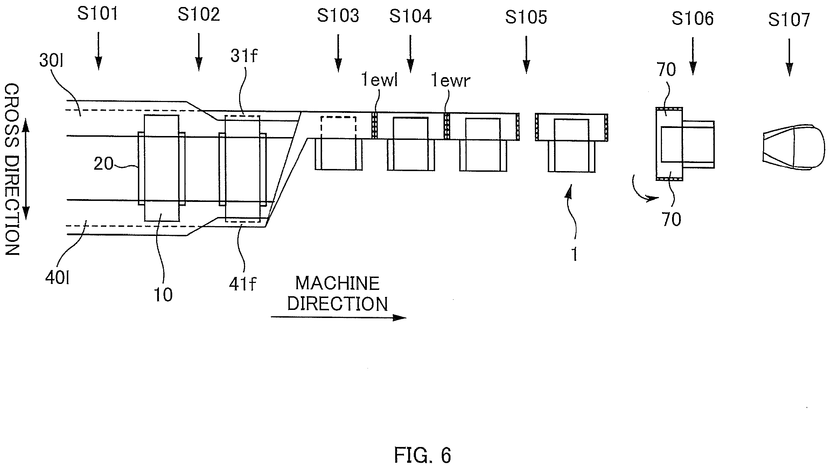

Description follows regarding an outline of a method for manufacturing the diaper 1. Fig. 5 is a diagram illustrating a manufacture flow for manufacturing the diaper 1. Fig. 6 is a schematic diagram to explain each of the processes when manufacturing the diaper 1. The diaper 1 of the present embodiment is manufactured by successively executing each of the processes from S101 to S107 illustrated in Fig. 5 on a manufacturing line.

First, base materials for the diaper 1 are conveyed at a predetermined conveyance speed along a predetermined conveying direction (S101). Reference to "base materials of the diaper 1" means a front band-shaped member 301 configured by plural front exterior members 30 in a state connected together in the lateral direction, and a back band-shaped member 401 configured by plural back exterior members 40 in a state connected together in the lateral direction. These members are respectively conveyed in a conveying direction along the lateral direction (width direction in Fig. 2) while a positional relationship of a predetermined gap in the height direction (the length direction in Fig. 2) is held between these members. Such a conveying state is also called a "widthwise flow state". Moreover, in the following description a direction along the conveying direction will be called the Machine Direction, and a direction orthogonal to the Machine Direction will be called the Cross Direction.

Next, the absorbent main bodies 10 and the leg-band members 20 are arranged so as to span across in the Cross Direction between the front band-shaped member 301 and the back band-shaped member 401 as they are being conveyed in the Machine Direction, and the absorbent main bodies 10 and the leg-band members 20 are joined thereto (S102) . In the process of S102, a height direction end portion of the front band-shaped member 301 (the exterior back sheet 31) is folded over from the outside toward the inside, forming the folded-over section 31f. Similarly, a height direction end portion of the back band-shaped member 401 (the exterior back sheet 41) is folded over from the outside toward the inside, forming a folded-over section 41f. Each type of elastic member, such as the stretchable sheet 34 and the waist-gather elastic members 35, are appropriately arranged and joined by the processes of S102 and S101.

The absorbent main body 10 is then folded at a Cross Direction central portion (CL10) thereof, such that the front band-shaped member 301 and the back band back band-shaped member 401 are superimposed on each other in the thickness direction (S103) . The front band-shaped member 301 and the back band back band-shaped member 401 are then joined together at positions corresponding to the front side edges 30es (or the back side edges 40es) where the two lateral direction end portions of the front exterior member 30 (or of the back exterior member 40) will be formed. The right join portion lewr and the left join portion 1ewl are thereby formed (S104) . The front band-shaped member 301 and the back band back band-shaped member 401 are then cut at the positions of the two lateral direction end portions of the diaper 1 (namely, at positions of the front side edges 30es), thereby cutting and separating individual diapers 1 from the band-shaped member (S105).

The conveying direction of the cut and separated diapers 1 is then changed at a predetermined location on the conveying path. Specifically, the diapers 1 being conveyed along their lateral directions are rotated by 90 degrees with respect to the conveying direction (Machine Direction) as illustrated in Fig. 6, so as to be conveyed along their height directions (S106). This conveying state is also called a "lengthwise flow state". Namely, through the process of S106, the diapers 1 that were being conveyed in the widthwise flow state are turned in direction so as to be conveyed in the lengthwise flow state. Note that although in the example of Fig. 6 the diapers 1 are turned in direction such that the crotch sides thereof are pointing in the conveying direction, the diapers 1 may be turned in direction such that the waist opening sides thereof are pointing in the conveying direction.

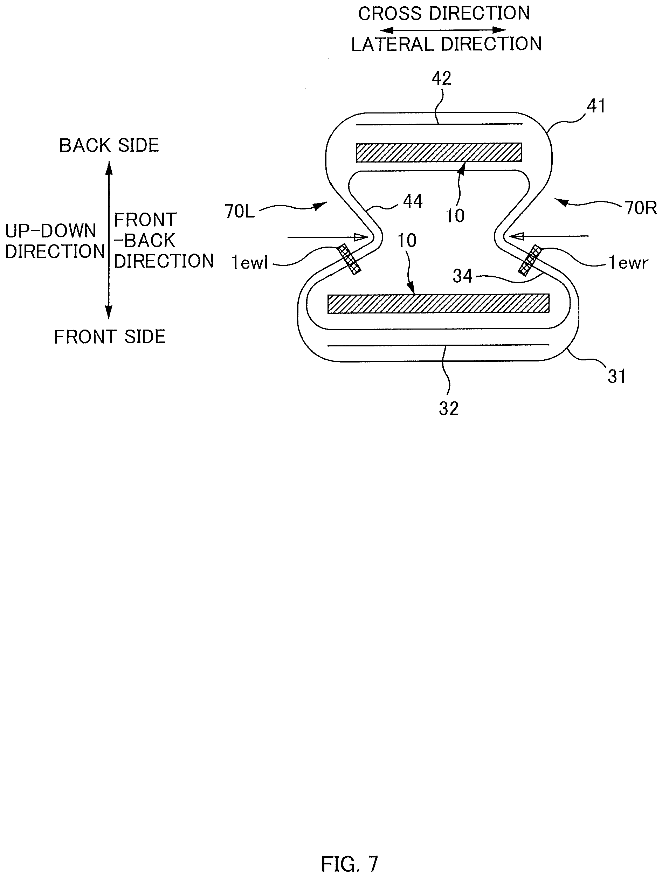

While the diapers 1 are being conveyed along their height directions, the side flaps 70L, 70R sticking out to the two lateral direction sides thereof are folded in from the outside toward the inside in the lateral direction (Cross Direction) (S107) . Fig. 7 is a cross-sectional view to explain a folding-in action performed on the side flaps 70. Fig. 7 illustrates a way in which the side flaps 70L, 70R are folded in inward in the lateral direction from the state illustrated in Fig. 4. In this folding-in process, respective regions in the vicinity of the up-down direction (front-back direction) center of the side flaps 70L, 70R are pushed inward from the outside toward the inside in the lateral direction, so as to be folded in between the front side and the back side of absorbent main body 10 in the up-down direction, while the side flaps 70L, 70R are being deformed into substantially Σ shapes, as illustrated in Fig. 7. The lateral direction size of the diapers 1 is thereby shortened, enabling compact packaging when shipping the manufactured diapers (product) 1. Moreover, when folding-in has been completed, the side flaps 70 are fixed in a state sandwiched between the back side and the front side of the absorbent main body 10. This means that the side flaps 70 are not liable to get in the way during packaging operations, and makes handling of the diapers 1 easy.

### <Side Flap Folding-In Action>

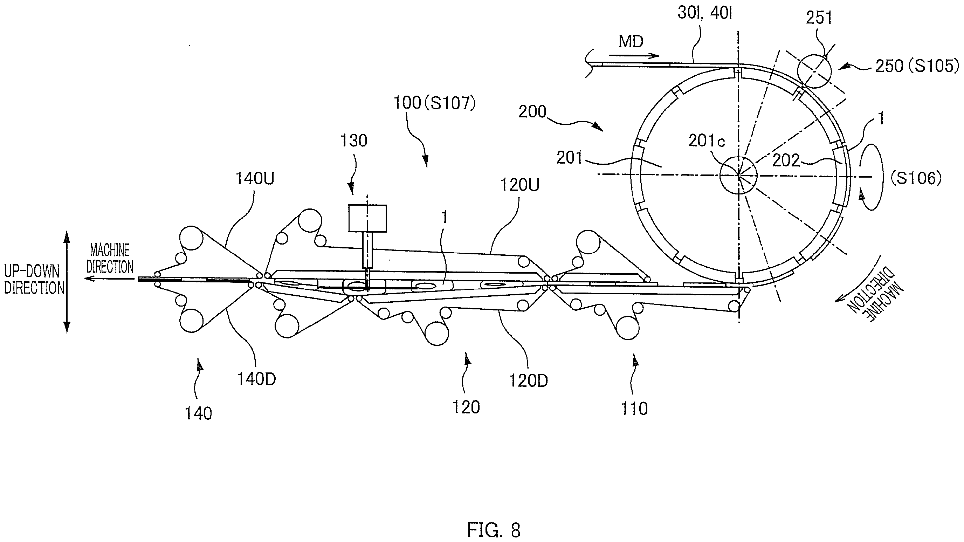

Details of the folding-in process of S107 of Fig. 5 as it is performed on the side flaps 70 will now be specifically described. Fig. 8 is a schematic side view illustrating an example of a configuration of a side flap fold-in mechanism 100. Fig. 8 illustrates, in an apparatus to manufacture the diapers 1, the side flap fold-in mechanism 100 that executes folding-in of the side flaps 70, and a turn drum 200 that conveys the diapers 1 and feeds the diapers 1 into the side flap fold-in mechanism 100.

Band-shaped base material (301, 401) for the diapers 1 that is continuous along the lateral direction is, in the process S101 to S105 described with reference to Fig. 5 and Fig. 6, guided onto the turn drum 200 while being conveyed in the widthwise flow state along the conveying direction (Machine Direction) by a predetermined conveying means such as a belt conveyor. The turn drum 200 is a conveying device that is capable of changing the orientation of the diapers 1 with respect to the conveying direction, and includes a rotation drum 201 and rotation pads 202. The rotation drum 201 is a drum shaped rotation body including a rotation shaft 201c provided parallel to the Cross Direction. The plural rotation pads 202 are supported at positions along the peripheral face of the rotation drum 201 and are rotated about the rotation shaft 201c in this supported state. The rotation pads 202 serve as holding sections that suction and hold the diapers 1 using a non-illustrated suction mechanism. The rotation pads 202 are rotatably supported on the peripheral face of the rotation drum 201 in a state in which the holding faces of the rotation pads 202 face toward the radial direction outside of the rotation drum 201. At the turn drum 200, the diapers 1 are conveyed along a rotation direction by rotation of the rotation drum 201 in a state in which the diapers 1 are held on the peripheral face of the turn drum 200 with the rotation pads 202 interposed therebetween. Namely, with the turn drum 200 the rotation direction of the rotation drum 201 is the conveying direction (Machine Direction).

The band-shaped base material (301, 401) of connected diapers 1 is cut using a cutter roll 250 while being conveyed with the turn drum 200 (S105). The cutter roll 250 is a rotating body arranged at a position facing toward the rotation drum 201. A cutter 251 is provided on the peripheral face of the cutter roll 250 so as to run along the axial direction of the cutter roll 250 (corresponding to the Cross Direction) . The base material of the diapers 1 is cut along the Cross Direction by opposing the cutter 251 to the peripheral face of the rotation drum 201, and by nipping the base material of the diapers 1 therebetween. The rotation pads 202 are then rotated by 90 degrees so as to be turned in direction such that the crotch side of the diapers 1 points in the conveying direction (Machine Direction), while each of the individual diapers 1 resulting from cutting the base material is held by the respective rotation pad 202 (S106) . Note that details regarding a specific mechanism and operation of the rotation drum 201 and the rotation pads 202 are disclosed in, for example, JP-A-2011-139862. Detailed description thereof is accordingly omitted. The diapers 1 are thereby fed by the turn drum 200 into the side flap fold-in mechanism 100 while being conveyed in a lengthwise flow state in which the height direction of the diapers 1 is aligned in the Machine Direction. Note that, as illustrated in Fig. 8, in the side flap fold-in mechanism 100, the diapers 1 are conveyed with their height direction along the conveying direction (Machine Direction) in a state in which the front-back direction of the diapers 1 is aligned with the up-down direction. Moreover, the turning in direction of the diapers 1 in the process of S106 may be performed using another mechanism other than the turn drum 200 described above.

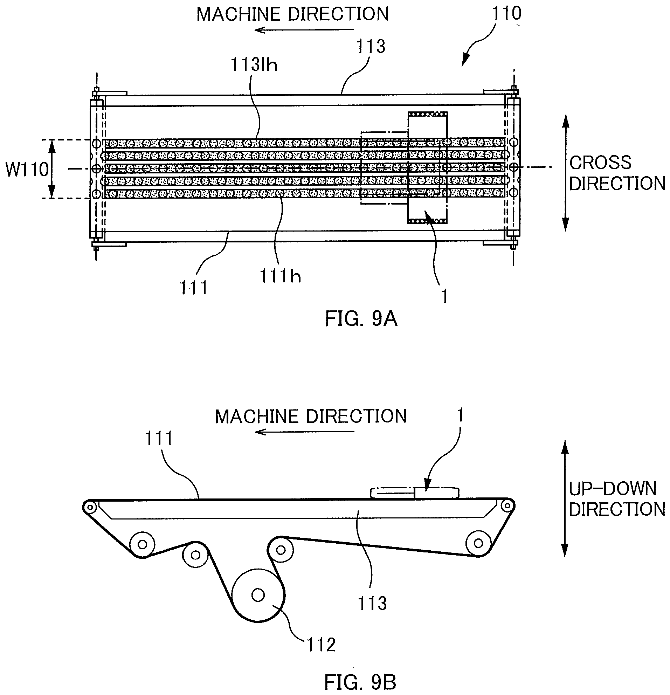

The side flap fold-in mechanism 100 includes the conveying means 110, a pulling means 120, the fold-in means 130, and a compressing means 140. The conveying means 110 has a function to convey the diapers 1 along the conveying direction (Machine Direction), and, for example, a suction conveyor may be employed therefor. Fig. 9A and Fig. 9B are diagrams to explain an example of a configuration of the conveying means 110. The conveying means 110 of the present embodiment includes a conveyor belt 111, a belt drive section 112, and a suction box 113. The conveyor belt 111 conveys the diapers 1 along the conveying direction (Machine Direction) by the conveyor belt 111 being driven by the belt drive section 112 while the diapers 1 are held on the belt surface. Plural suction holes 111h are provided in the surface of the conveyor belt. The suction holes 111h are round holes of a predetermined size, and are provided so as to span the entire length direction (namely, direction along the Machine Direction) of the conveyor belt 111. The suction holes 111h are provided so as to span a range of a predetermined width W110 in the Cross Direction of the conveyor belt 111. The suction box 113 includes plural suction grooves 1131h (illustrated by shading in Fig. 9A) running along the Machine Direction, and external air is sucked into the suction box 113 through the suction grooves 1131h. Suctioning in the conveying means 110 is accordingly performed in the region where the suction grooves 1131h of the suction box 113 overlap with the suction holes 111h of the conveyor belt 111, as illustrated in Fig. 9A. The diapers 1 are accordingly conveyed along the Machine Direction in a state held onto the surface of the conveyor belt 111 by suction.

The pulling means 120 includes a function to open the waist opening 1HB (the side flaps 70) out in the front-back direction by pulling a front region and a back region of the absorbent main bodies 10 toward opposite sides in the up-down direction (the front-back direction of the diapers 1) as the diapers 1 are being conveyed along the Machine Direction. For the pulling means 120, a suction conveyor similar to the conveying means 110 may, for example, be employed. In the example of the pulling means 120 in Fig. 8, an upward suction conveyor 120U and a downward suction conveyor 120D are provided so as to be arranged facing toward each other in the up-down direction. The upward suction conveyor 120U and the downward suction conveyor 120D each include configuration substantially the same as that of the conveying means 110 illustrated in Fig. 9A and Fig. 9B. Namely, the upward suction conveyor 120U and the downward suction conveyor 120D respectively include conveyor belts 121U, 121D, belt drive sections 122U, 122D, and suction boxes 123U, 123D. The diapers 1 are then conveyed in a state in which the upward suction conveyor 120U suctions and holds a portion of the back region of each of the absorbent main bodies 10 upward, and the diapers 1 are conveyed in a state in which the downward suction conveyor 120D suctions and holds a portion at the front region of each of the absorbent main bodies 10 downward. Note that upward suction conveyor 120U and the downward suction conveyor 120D are configured to enable the diapers 1 to be respectively conveyed at the same speed along the Machine Direction.

In the present embodiment, the gap in the up-down direction between the upward suction conveyor 120U and the downward suction conveyor 120D widens on progression downstream in the conveying direction (Machine Direction) . Specifically, as illustrated in Fig. 8, the conveying face of the downward suction conveyor 120D is arranged so as to be inclined with respect to the conveying face of the upward suction conveyor 120U. Thus the front region and the back region of the absorbent main body 10 are each pulled toward opposite sides in the up-down direction while the diapers 1 are being conveyed along the Machine Direction, thereby opening the waist opening 1HB formed by the side flaps 70 upward and downward (toward the front and back of the diapers 1) . Note that the front-back direction of the diapers 1 as they are being conveyed may be the opposite to the situation described above. Namely, the front region of the absorbent main body 10 may be pulled upward, and the back region of the absorbent main body 10 may be pulled downward.

Moreover, as described above, due to the elasticity in the lateral direction being different in the diapers 1 at the front elastic region ERf to at the back elastic region ERb, the size and shape of creases that develop on the surfaces of the front exterior member 30 and the back exterior member 40 are different on the back side and the front side. Thus at the lateral direction outside of the absorbent main body 10, the front exterior member 30 and the back exterior member 40 (namely, the side flaps 70) that are overlapped with each other in the front-back direction (up-down direction) are not liable to stick together due to the effect of such creases. This means that the front exterior member 30 and the back exterior member 40 of the side flaps 70 are easily separated from each other when the diapers 1 are pulled in the up-down directions by the pulling means 120, facilitating the upward/downward opening of the waist opening 1HB. Namely, the front exterior member 30 and the back exterior member 40 are not susceptible to a problem of them sticking together and becoming difficult to separate upward/downward.

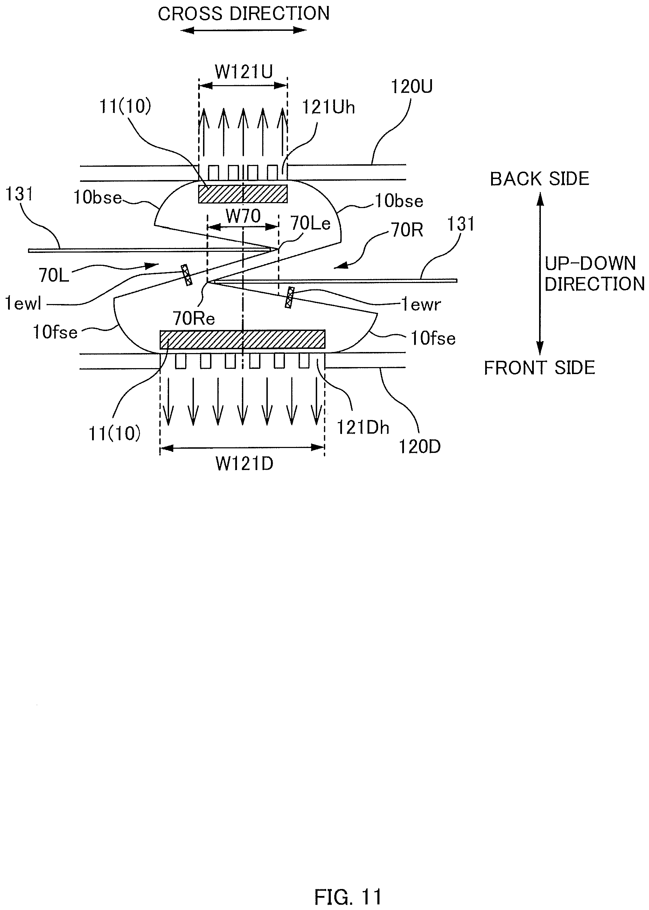

Moreover, in the upward suction conveyor 120U, plural suction holes 121Uh are provided over a range of width W121U in the Cross Direction of the conveyor belt 121U, and a portion of the back region of the absorbent main body 10 is pulled upward in the range of the width W121U (see Fig. 11) . Similarly, in the downward suction conveyor 120D, plural suction holes 121Dh are provided over a range of width W121D in the Cross Direction of the conveyor belt 121D, and a portion of the back region of the absorbent main body 10 is pulled upward in the range of the width W121D (see Fig. 11) . The width W121D is wider than the width W121U (W121D > W121U) . As described above, the absorbent main body 10 of the present embodiment more readily contracts in the lateral direction at the back side than at the front side, such that the lateral direction width of portions at the back side of the absorbent main body 10 is narrower than the lateral direction width of portions at the front side thereof. Thus by setting a suction range (W121D) at the front side different to a suction range (W121U)at the back side to match the lateral direction widths of the absorbent main body 10 after contraction, the absorbent main body 10 can be stably pulled in the up-down directions without being susceptible to positional misalignment or the like.

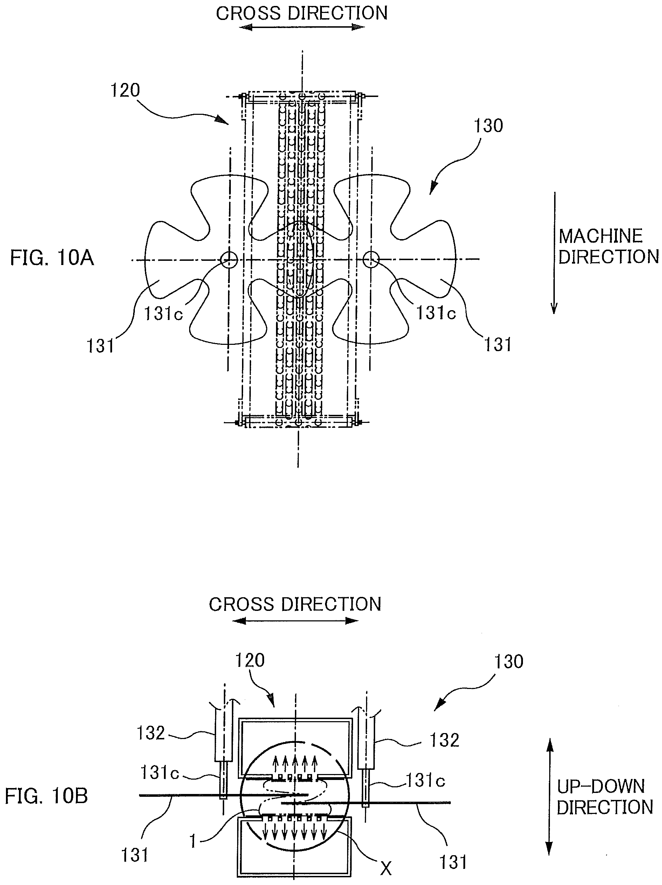

The fold-in means 130 has a function to respectively fold the side flaps 70L, 70R of the diapers 1 that have been opened out in the front-back direction (up-down direction) from the outside toward the inside in the lateral direction (Cross Direction) and, for example, an impeller may be employed therefor. Fig. 10A and Fig. 10B are diagrams to explain an example of a configuration of the fold-in means 130. Fig. 11 is a diagram illustrating an enlargement of region X in Fig. 10B. The fold-in means 130 of the present embodiment includes impellers 131 and impeller drive sections 132. The impellers 131 are configured by a pair of impellers provided on both Cross Direction sides of the pulling means 120, and each of the impellers rotates about a rotation center of a rotation shaft 131c disposed along the up-down direction. The impeller drive sections 132 are drive sections to rotationally drive the impellers 131.

As illustrated in Fig. 8, the fold-in means 130 is provided downstream of the pulling means 120 in the conveying direction. The rotationally driven impellers 131 push against the side flaps 70L, 70R of the diapers 1, now in a sufficiently opened state in the front-back direction (up-down direction) due to the pulling means 120, by pushing inward in the lateral direction (Cross Direction) from the outside toward the inside. The left and right side flaps 70L, 70R are thereby respectively deformed into Σ shapes as illustrated in Fig. 10B, facilitating the holding of a constant folded-in amount when the absorbent main body 10 is folded in between the front side and the back side thereof (namely, somewhere along the front-back direction). The diapers 1 are thereby easily folded into a uniform shape.

In the fold-in means 130 of the present embodiment, the left and right impellers 131 provided at each Cross Direction side are arranged so their heights in the up-down direction are at different positions to each other. As illustrated in Fig. 11, in a state in which the side flaps 70L, 70R have been folded in the lateral direction (Cross Direction) to the furthest extent toward the inside, the right impeller 131 and the left impeller 131 are arranged such that portions of the respective impellers 131 partially overlap in the up-down direction. Such an arrangement enables the side flaps 70L, 70R to be folded inward more deeply. Specifically, when the left side flap 70L has been folded inward to the maximum extent in the lateral direction (Cross Direction), the left side flap 70L is folded inward deeply to the extent that a fold edge 70Le is positioned on the right side of a central position in the Cross Direction. Similarly, when the right side flap 70R has been folded inward to the maximum extent in the lateral direction (Cross Direction), the right side flap 70R is folded inward deeply to the extent that a fold edge 70Re is positioned on the left side of the central position in the Cross Direction. As a result of this, an overlap region W70 is formed where portions of the folded in left side flap 70L and right side flap 70R overlap with each other in the front-back direction (up-down direction) . Frictional force arises in the overlap region W70 due to mutual contact between the left side flap 70L and right side flap 70R that are overlapped in the front-back direction (up-down direction). These overlapped portions are accordingly not readily separated. Thus, even when the diapers 1 are conveyed further downstream than the position of the fold-in means 130, and the pushing in action by the impellers 131 toward the lateral direction inside no longer acts, the folded-in state of the side flaps 70 is easily maintained.

Note that the height in the up-down direction of the impellers 131 at the two Cross Direction sides is adjusted so as to enable a folding-in action to be performed smoothly on the side flaps 70L, 70R, and so as to facilitate the generation of friction when the side flaps 70L, 70R are in the folded-in state by causing the side flaps 70L, 70R to contact each other in the up-down direction in the overlap region W70.

Due to the side flaps 70L, 70R being folded deeply inwards in the lateral direction (Cross Direction) in this manner, the end portions at the two lateral direction sides of the absorbent main body 10 are pulled in by the side flaps 70L, 70R and deformed so as to bend toward the inside in the up-down direction. In Fig. 11, at the back side of the absorbent main body 10, the two lateral direction end portions 10bse bend downward in the up-down direction so as to separate from the upward suction conveyor 120U (the conveyor belt 121U) . Similarly, at the front side of the absorbent main body 10, the two lateral direction end portions 10fse bend upward in the up-down direction so as to separate from the downward suction conveyor 120D (the conveyor belt 121D). Note that although in Fig. 11 the two lateral direction end portions 10bse, 10fse of the absorbent main body 10 are illustrated in a curved state so as to bend in a bowed shape, cases in which the two lateral direction end portions 10bse, 10fse bend at an acute angle instead of bending into a bowed shape are also encompassed by the term "curved". The width in the lateral direction of the diapers 1 is smaller due to causing the two lateral direction end portions of the absorbent main body 10 to be curved, thereby enabling the diapers 1 to be packaged more compactly.

Note that a state in which "the lateral direction end portions of the absorbent main body 10 are curved" also encompasses cases in which the lateral direction end portions of the absorbent core 11 are curved. Deeply folding the side flaps 70L, 70R respectively inward in the lateral direction to the extent that the lateral direction end portions of the absorbent core 11 are curved facilitates forming the overlap region W70 described above, and makes it easy to maintain a folded-in state. It also enables the diapers 1 to be folded more compactly.

Moreover, the impellers 131 are adjusted in the up-down direction so that the left join portion 1ewl and the right join portion lewr provided on the side flaps 70 are folded in to different positions. This achieves, as illustrated in Fig. 11, a state in which the positions of the fold edge 70Le and the left join portion 1ewl of the left side flap 70L are offset from each other in the up-down direction, and the positions of the fold edge 70Re and the right join portion lewr of the right side flap 70R are offset from each other in the up-down direction. Due to the left and right join portions lewl, lewr having a predetermined thickness due to welding or the like, sometimes problems arise if the join portions lewl, lewr are arranged so as to respectively overlap with the fold edges 70Le, 70Re. For example, there is a concern regarding the thickness arising at the folded-in portions, difficulties in forming a fold line at the fold edges 70Le, 70Re, and difficulties in maintaining the folded-in state. However, in the present embodiment, such problems are not liable to arise due to the position of the join portions lewl, lewr and the position of the fold edges 70Le, 70Re being offset from each other.

Furthermore, in the present embodiment, the side flaps 70 are folded in such that the positions of the join portions lewl, lewr do not overlap with the overlap region W70 in the front-back direction (up-down direction) (see Fig. 11) . The front-back direction thickness is greater at the overlap region W70 due to the plural materials that overlap with each other in the front-back direction (up-down direction) . Thus in cases in which the join portions lewl, lewr are formed in the overlap region W70, the overlap region W70 has an even greater thickness due to the certain thickness of the join portions lewl, lewr themselves, making it difficult to package the diapers 1 compactly. In contrast thereto, the thickness of the diapers 1 is suppressed from increasing in the front-back direction by configuring such that the join portions lewl, lewr do not overlap with the overlap region W70.

The diapers 1 that have side flaps 70 folded in by the fold-in means 130 are then compressed in the front-back direction (up-down direction) by the compressing means 140. The compressing means 140 has functionality to compress the diapers 1 in the front-back direction (up-down direction) by nipping the entirety of the diapers 1 from above and below as the diapers 1 are being conveyed along the Machine Direction. A belt conveyor may, for example, be employed as the compressing means 140. In the example of Fig. 8, an upper belt conveyor 140U and a lower belt conveyor 140D arranged so as to face each other in the up-down direction are provided as the compressing means 140. The upper belt conveyor 140U and the lower belt conveyor 140D respectively include conveyor belts 141U, 141D and belt drive sections 142U, 142D. A gap h140 in the up-down direction between the conveyor belts 141U, 141D is maintained at a predetermined size, and the diapers 1 are compressed in the up-down direction by being nipped between the upper belt conveyor 140U and the lower belt conveyor 140D (in the gap h140).

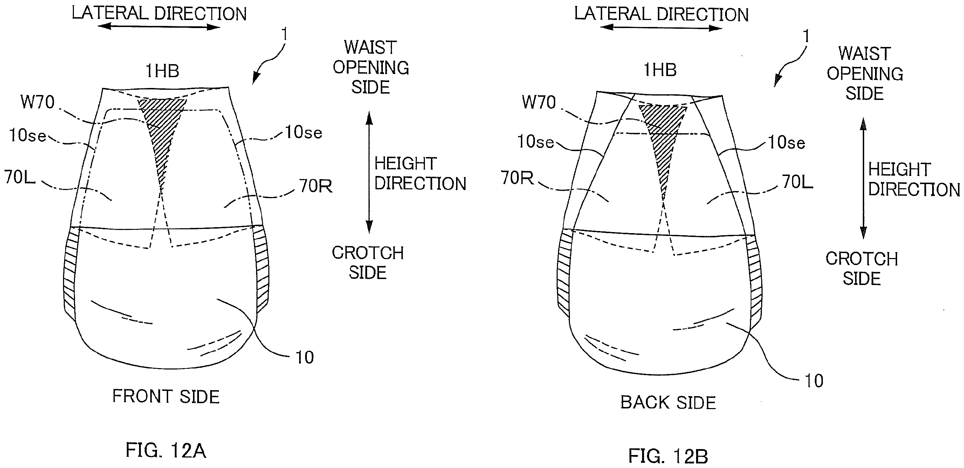

Fig. 12A is a schematic plan view of the diaper 1 in a state in which the side flaps 70 have been folded in, as viewed from the front side. Fig. 12B is a schematic plan view of the diaper 1 in a state in which the side flaps 70 have been folded in, as viewed from the back side. The region illustrated by shading in Fig. 12A and Fig. 12B is the overlap region W70 where the side flaps 70L, 70R that have been folded in are overlapped with each other in the front-back direction.

The front elastic region ERf is formed at the front side of the diapers 1 by the stretchable sheet 34 in the region at the height direction upper side of the absorbent main body 10, and contraction force in the lateral direction readily acts in this upper side region (see Fig. 2). This results in a shape in which, as illustrated in Fig. 12A, the absorbent main body 10 (the absorbent core 11) is contracted in the lateral direction at the height direction upper side. Then, due to the side flaps 70L, 70R being folded inward in the lateral direction along the profile of the two lateral direction side end portions lOse of the absorbent main body 10 (the absorbent core 11) , the side flaps 70L, 70R are more readily folded deeply inward in the lateral direction at the height direction upper end side than at the height direction lower end side thereof. Namely, the surface area of portions on the waist opening side where the side flaps 70 are folded in between the absorbent main body in the up-down direction is greater than the surface area of portions on the crotch side where the side flaps 70 are folded in between the absorbent main body in the up-down direction. As a result, the overlap region W70 is formed at the height direction upper end side (waist opening side) of the side flaps 70L, 70R, as illustrated in Fig. 12A, and the overlap region W70 is not formed at the height direction lower end side (the crotch side) .

When the side flaps 70L, 70R are folded in, the side flaps 70L, 70R are not caused to overlap over the entire region in the height direction, and instead are only caused to overlap in a part region in the height direction. This suppresses an increase in thickness (width in the front-back direction) of the diapers 1 as a whole, thereby facilitating compact packaging thereof. Moreover, due to compressing the diapers 1 using the compressing means 140 after the side flaps 70L, 70R have been folded in, the frictional force arising between the side flap 70L and the side flap 70R is greater where they are overlapped in the overlap region W70, rendering it more difficult to separate the side flaps 70L, 70R from each other. In particular, due to it being easier to maintain a folded-in state of the side flaps 70L, 70R at the waist opening side of the diapers 1, the waist opening 1HB is suppressed from inadvertently opening during packaging operations or the like.

Moreover, the back elastic region ERb is formed at the back side of the diapers 1 by the stretchable sheet 44 in the region at the height direction upper side of the absorbent main body 10, and contraction force in the lateral direction readily acts in this upper side region (see Fig. 2) . Thus, similarly to at the front side, the side flaps 70L, 70R at the back side are also folded in deeply inward in the lateral direction more readily at the height direction upper side than at the lower side thereof. Moreover, due to a larger contraction force acting at the back elastic region ERb than at the front elastic region ERf, the absorbent main body 10 is contracted more in the lateral direction at the back side than at the front side. This means that, as illustrated in Fig. 12B, the side flaps 70L, 70R are folded in to positions more inward in the lateral direction at the back side of the diapers 1 than at the front side thereof. Thus when the diapers 1 are viewed with the side flaps 70 in the folded-in state, the profiles are clearly different from each other at the front side and the back side, enabling a user to easily discern the front and back (the front side and the back side) of the diapers 1. Note that the absorbent main body 10 may be configured so as to be contracted more in the lateral direction at the front side than at the back side, with the front and back of the diapers 1 also being easily discerned in such cases.

### <Modified Example>

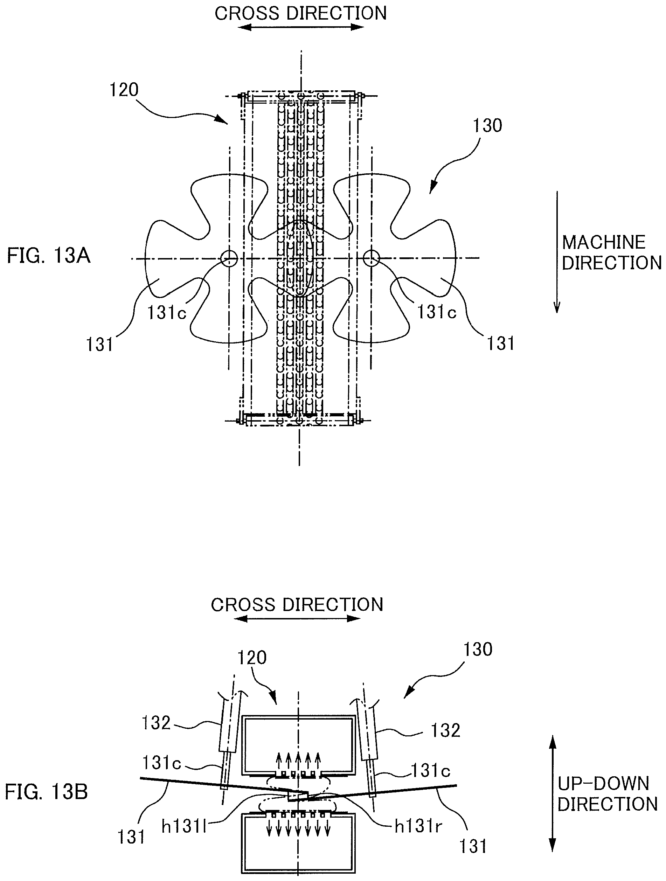

Fig. 13A and Fig. 13B are diagrams illustrating a modified example of the fold-in means 130. In this modified example, rotation shafts 131c of the impellers 131 arranged at each Cross Direction side are respectively arranged so as to be inclined at a predetermined angle toward the outside in the lateral direction, and the side flaps 70 are folded in as the inclined impellers 131 rotate. Other configuration and functions are basically the same as those of the fold-in means 130 described with reference to Fig. 10A and Fig. 10B.

A gap in the up-down direction between the impellers 131 is different according to position at an overlap portion where the right impeller 131 and the left impeller 131 overlap in the lateral direction (Cross Direction) , due to the left and right impellers 131 rotating in an inclined state relative to a horizontal plane. In Fig. 13B, an up-down direction gap h131r is shortest between a right end portion of the left impeller 131 and the right impeller 131, and an up-down direction gap h1311 is longest between a left end portion of the right impeller 131 and the left impeller 131. Inward folding of the side flaps 70 can be performed smoothly due to the up-down direction gap between the impellers 131 varying in this manner. For example, when the side flaps 70L, 70R are being folded in, a smooth action of the impellers 131 is easily achieved due to the side flaps 70L, 70R not interfering with each other in the region of the gap h1311. Moreover, due to portions of the side flaps 70L, 70R at the gap h131r being compressed the up-down direction by the impellers 131 and frictional force arising, the side flaps 70 when folded-in are not readily separated from each other.

Note that a configuration may be adopted in which the impellers 131 arranged on the Cross Direction sides are not both arranged at an inclination, and only a rotation shaft 131c of the impeller 131 on one side is inclined, with the rotation shaft 131c of the impeller 131 on the other side arranged vertically along the up-down direction.

Moreover, the fold-in means 130 may be configured by an airflow pressure style of fold-in means. A blow pressure style of fold-in means 130 includes blow nozzles and a blower (neither of which is illustrated in the drawings) . The blow nozzles are provided in place of the impellers, and compressed air is ejected from the blow nozzles from a lateral direction outside toward the lateral direction inside. The side flaps 70 are thereby folded inward in the lateral direction by the air being blown against the side flaps 70 of the diapers 1 as they are being conveyed along the Machine Direction. Due to impellers not being needed when a blow pressure style of fold-in means is employed, damage to the side flaps 70 due to contact with tip portions of the impellers can be suppressed from occurring.

### ===Other Embodiments===

The embodiments described above are merely to facilitate understanding of the invention, and are not meant to be interpreted in a manner limiting the scope of the invention. The invention can of course be modified and improved without departing from the gist thereof and the invention includes functional equivalents of such modifications and improvements. For example, the following modifications may be made.

In the embodiment described above, an example is described in which a nonwoven fabric is employed as the material configuring the exterior back sheet 31 and the exterior back sheet 41. However, the material configuring each of these portions is not limited to such a nonwoven fabric. For example, a woven fabric may be employed therefor, or a sheet member that is not a woven fabric may be employed therefor.

In the embodiment described above, an example is described in which rubber threads are employed as the waist-gather elastic members 35 and the fit-gather elastic members 36. However, there is no limitation thereto. For example, band-shaped rubber may be employed therefor, or an elastic band-shaped nonwoven fabric or an elastic band-shaped resin film may be employed therefor.

### Reference Signs List

1: diaper (absorbent article, pull-on disposable diaper);
lewr: right join portion; 1ewl: left join portion;
1HB: waist opening; 1HL: leg opening;
10: absorbent main body;
101e: end portions (height direction); 10se: end portion (lateral direction);
10fse end portion (lateral direction/front side); 10bse: end portion (lateral direction/ back side);
11: absorbent core;
13: front-face sheet; 15: back-face sheet; 15a: leak prevention sheet;
15b: exterior sheet;
20: leg-band member; 201e: end portion (height direction); 30: front exterior member;
30es: front side edge; 301: front band-shaped member;
31: exterior back sheet; 31f: folded-over section;
32: film sheet; 33: cover sheet; 34: stretchable sheet;
35: waist-gather elastic member; 36: fit-gather elastic member;
40: back exterior member;
40es: back side edge; 401: back band-shaped member; 41: exterior back sheet; 41f: folded-over section;
42: film sheet; 43: cover sheet; 44: stretchable sheet;
45: waist-gather elastic member; 46: fit-gather elastic member;
70: side flap;
70R: right side flap; 70Re: fold edge;
70L: left side flap; 70Le: fold edge;
100: side flap fold-in mechanism;
110: conveying means;
111: conveyor belt; 111h: suction hole; 112: belt drive section;
113: suction box; 1131h: suction groove;
120: pulling means;
120U: upward suction conveyor; 121U: conveyor belt;
121Uh: suction hole; 122U: belt drive section; 123U suction box;
120D: downward suction conveyor; 121D: conveyor belt;
121Dh: suction hole; 122D belt drive section; 123D suction box;
130: fold-in means;
131: impeller; 131c: rotation shaft; 132: impeller drive section;
140: compressing means;
140U: upper belt conveyor;
141U: conveyor belt; 142U: belt drive section;
140D: lower belt conveyor;
141D: conveyor belt; 142D belt drive section;
200: turn drum;
201: rotation drum; 201c: rotation shaft; 202: rotation pad; 250: cutter roll; 251: cutter;
W70: overlap region;
h131r: gap; h1311: gap;
CL10: predetermined position;
ERf: front elastic region; ERf: front elastic region;
LG leg gather;
LSG: barrier cuff.

## Claims

1. A method for manufacturing an absorbent article (1) for manufacturing a pull-on absorbent article (1) having a height direction, a lateral direction, and a front-back direction intersecting with each other, the absorbent article (1) including an absorbent main body (10) configured to absorb excrement, a front exterior member (30) arranged at one end side of the absorbent main body (10), and a back exterior member (40) arranged at another end side of the absorbent main body, with the front exterior member (30) and the back exterior member (40) joined together at a pair of join portions, the method for manufacturing an absorbent article (1) comprising:
in a state in which the front-back direction of the pull-on absorbent article (1) is aligned in an up-down direction and the pull-on absorbent article (1) is being conveyed in a conveying direction along the height direction,
pulling at least a portion on the front side of the absorbent main body (10) toward one side in the up-down direction and pulling at least a portion on the back side of the absorbent main body (10) toward the other side in the up-down direction; and
folding in the front exterior member (30) and the back exterior member (40) between the front side and the back side of the absorbent main body (10) in the up-down direction until an end portion in the lateral direction at the front side of the absorbent main body (10) is curved toward the other side in the up-down direction and an end portion in the lateral direction at the back side of the absorbent main body (10) is curved toward the one side in the up-down direction, wherein
in the folding,
the front exterior member (30) and the back exterior member (40) are folded in between the front side and the back side of the absorbent main body (10) such that a surface area of a folded-in portion of the front exterior member (30) and the back exterior member (40) folded in between the front side and the back side of the absorbent main body (10) in the up-down direction at a waist opening side in the height direction is larger than a surface area of a folded-in portion of the front exterior member (30) and the back exterior member (40) folded in between the front side and the back side of the absorbent main body (10) in the up-down direction at a crotch side in the height direction.

2. The method for manufacturing an absorbent article of claim 1, wherein:
when the front exterior member (30) and the back exterior member (40) are folded in, a portion of overlap in the up-down direction is made where the front exterior member (30) and the back exterior member (40) arranged at one side in the lateral direction overlap with the front exterior member (30) and the back exterior member (40) arranged at another side in the lateral direction.

3. The method for manufacturing an absorbent article of claim 2, wherein:
at the overlapping portion in the up-down direction where the front exterior member (30) and the back exterior member (40) arranged at the one lateral direction side overlap with the front exterior member (30) and the back exterior member (40) arranged at the other lateral direction side,
the front exterior member (30) and the back exterior member (40) are folded inward such that the front exterior member (30) and the back exterior member (40) arranged at the one lateral direction side contact the front exterior member (30) and the back exterior member (40) arranged at the other lateral direction side.

4. The method for manufacturing an absorbent article of any one of claim 1 to claim 3, wherein:
the absorbent main body (10) contracts in the lateral direction due to an elastic region that stretches and contracts along the lateral direction; and
an amount of contraction in the lateral direction of the absorbent main body (10) at the back side is different to an amount of contraction in the lateral direction of the absorbent main body (10) at the front side.

5. The method for manufacturing an absorbent article of claim 4, wherein:
a surface area of a portion where the absorbent main body (10) is pulled toward the one side in the up-down direction at the front side is different to a surface area of a portion where the absorbent main body (10) is pulled toward the other side in the up-down direction at the back side.

6. The method for manufacturing an absorbent article of any one of claim 1 to claim 5, wherein:
the front exterior member (30) and the back exterior member (40) are folded in between the front side and the back side of the absorbent main body (10) in the up-down direction by a pair of impellers respectively provided at each side of the pull-on absorbent article (1) in a direction intersecting the conveying direction; and
the pair of impellers include a portion where the impellers overlap with each other in the up-down direction.

7. The method for manufacturing an absorbent article of claim 6, wherein:
the front exterior member (30) and the back exterior member (40) are folded in between the front side and the back side of the absorbent main body (10) in the up-down direction by the pair of impellers pushing against positions on the front exterior member (30) and the back exterior member (40) different from the positions of the join portions.

8. The method for manufacturing an absorbent article of claim 6 or claim 7, wherein:
a rotation shaft of at least one impeller out of the pair of impellers is arranged at a predetermined angle of inclination to the up-down direction.

9. The method for manufacturing an absorbent article of any one of claim 1 to claim 5, wherein:
the front exterior member (30) and the back exterior member (40) are folded in between the front side and the back side of the absorbent main body (10) in the up-down direction by ejecting air from a pair of blow nozzles respectively provided at each side of the pull-on absorbent article (1) in a direction intersecting the conveying direction so as to blow the air against the front exterior member (30) and the back exterior member.

10. The method for manufacturing an absorbent article of any one of claim 1 to claim 9, wherein the method for manufacturing an absorbent article includes:
a process in which a front band-shaped member configured by a plurality of the front exterior members connected together contiguously along the lateral direction, and a back band-shaped member configured by a plurality of the back exterior members connected together contiguously along the lateral direction, are conveyed along the lateral direction, and while being conveyed along the lateral direction the front band-shaped member and the back band-shaped member are cut at respective end portions in the lateral direction of the front exterior members and the back exterior members; and
a process in which a direction in which the front band-shaped member and the back band-shaped member are being conveyed is turned around by a predetermined angle so that when they have been cut the cut front band-shaped members and the cut back band-shaped members are conveyed along the height direction.

## Patentansprüche

1. Verfahren zur Herstellung eines saugfähigen Artikels (1) zur Herstellung eines überziehbaren saugfähigen Artikels (1), der eine Höhenrichtung, eine seitliche Richtung und eine Vorwärts-Rückwärtsrichtung aufweist, die einander schneiden, wobei der saugfähige Artikel (1) einen saugfähigen Hauptkörper (10), der zum Aufsaugen von Exkrement konfiguriert ist, einen vorderes äußeres Element (30), das an einer Stirnseite des saugfähigen Hauptkörpers (10) angeordnet ist, und ein hinteres äußeres Element (40), das an einer anderen Stirnseite des saugfähigen Hauptkörpers angeordnet ist, beinhaltet, wobei das vordere äußere Element (30) und das hintere äußere Element (40) an einem Paar von Vereinigungsabschnitten zusammengefügt sind, wobei das Verfahren zur Herstellung eines saugfähigen Artikels (1) Folgendes umfasst:
in einem Zustand, in welchem die Vorwärts-Rückwärtsrichtung des überziehbaren saugfähigen Artikels (1) in einer Aufwärts-Abwärtsrichtung ausgerichtet ist und der überziehbare saugfähige Artikel (1) in einer Förderrichtung entlang der Höhenrichtung befördert wird,
Ziehen mindestens eines Abschnitts auf der Vorderseite des saugfähigen Hauptkörpers (10) in Richtung einer Seite in der Aufwärts-Abwärtsrichtung und Ziehen mindestens eines Abschnitts auf der Rückseite des saugfähigen Hauptkörpers (10) in Richtung der anderen Seite in der Aufwärts-Abwärtsrichtung; und
Einklappen des vorderen äußeren Elements (30) und des hinteren äußeren Elements (40) zwischen der Vorderseite und der Rückseite des saugfähigen Hauptkörpers (10) in der Aufwärts-Abwärtsrichtung, bis ein Stirnabschnitt in der seitlichen Richtung an der Vorderseite des saugfähigen Hauptkörpers (10) in Richtung der anderen Seite in der Aufwärts-Abwärtsrichtung gekrümmt ist und ein Stirnabschnitt in der seitlichen Richtung an der Rückseite des saugfähigen Hauptkörpers (10) in Richtung der einen Seite in der Aufwärts-Abwärtsrichtung gekrümmt ist, wobei das vordere äußere Element (30) und das hintere äußere Element (40) beim Einklappen zwischen der Vorderseite und der Rückseite des saugfähigen Hauptkörpers (10) derart eingeklappt sind, dass eine Oberfläche eines eingeklappten Abschnitts des vorderen äußeren Elements (30) und des hinteren äußeren Elements (40), die zwischen der Vorderseite und der Rückseite des saugfähigen Hauptkörpers (10) in der Aufwärts-Abwärtsrichtung an einer Taillenöffnungsseite in der Höhenrichtung eingeklappt sind, größer ist als eine Oberfläche eines eingeklappten Abschnitts des vorderen äußeren Elements (30) und des hinteren äußeren Elements (40), die zwischen der Vorderseite und der Rückseite des saugfähigen Hauptkörpers (10) in der Aufwärts-Abwärtsrichtung an einer Schrittseite in der Höhenrichtung eingeklappt sind.

2. Verfahren zur Herstellung eines saugfähigen Artikels nach Anspruch 1, wobei:
wenn das vordere äußere Element (30) und das hintere äußere Element (40) eingeklappt sind, ein Überlappungsabschnitt in der Aufwärts-Abwärtsrichtung dort hergestellt ist, wo sich das vordere äußere Element (30) und das hintere äußere Element (40), die auf einer Seite in der seitlichen Richtung angeordnet sind, mit dem vorderen äußeren Element (30) und dem hinteren äußeren Element (40), die auf einer anderen Seiten in der seitlichen Richtung angeordnet sind, überlappen.

3. Verfahren zur Herstellung eines saugfähigen Artikels nach Anspruch 2, wobei:
bei dem Überlappungsabschnitt in der Aufwärts-Abwärtsrichtung, wo sich das vordere äußere Element (30) und das hintere äußere Element (40), die auf der einen Seite der seitlichen Richtung angeordnet sind, mit dem vorderen äußeren Element (30) und dem hinteren äußeren Element (40), die auf der anderen Seite der seitlichen Richtung angeordnet sind, überlappen,
das vordere äußere Element (30) und das hintere äußere Element (40) derart nach innen geklappt sind, dass das vordere äußere Element (30) und das hintere äußere Element (40), die auf der einen Seite der seitlichen Richtung angeordnet sind, das vordere äußere Element (30) und das hintere äußere Element (40), die auf der anderen Seite der seitlichen Richtung angeordnet sind, berühren.

4. Verfahren zur Herstellung eines saugfähigen Artikels nach einem von Anspruch 1 bis Anspruch 3, wobei:
sich der saugfähige Hauptkörper (10) aufgrund einer elastischen Region, die sich entlang der seitlichen Richtung ausdehnt und zusammenzieht, in der seitlichen Richtung zusammenzieht; und
sich ein Ausmaß des Zusammenziehens in der seitlichen Richtung des saugfähigen Hauptkörpers (10) an der Rückseite von einem Ausmaß des Zusammenziehens in der seitlichen Richtung des saugfähigen Hauptkörpers (10) an der Vorderseite unterscheidet.

5. Verfahren zur Herstellung eines saugfähigen Artikels nach Anspruch 4, wobei:
sich eine Oberfläche eines Abschnitts, wo der saugfähige Hauptkörper (10) in Richtung der einen Seite in der Aufwärts-Abwärtsrichtung an der Vorderseite gezogen wird, von einer Oberfläche eines Abschnitts, wo der saugfähige Hauptkörper (10) in Richtung der anderen Seite in der Aufwärts-Abwärtsrichtung an der Rückseite gezogen wird, unterscheidet.

6. Verfahren zur Herstellung eines saugfähigen Artikels nach einem von Anspruch 1 bis Anspruch 5, wobei:
das vordere äußere Element (30) und das hintere äußere Element (40) zwischen der Vorderseite und der Rückseite des saugfähigen Hauptkörpers (10) in der Aufwärts-Abwärtsrichtung durch ein Paar von Laufrädern eingeklappt sind, die jeweils an jeder Seite des überziehbaren saugfähigen Artikels (1) in einer Richtung bereitgestellt sind, die die Förderrichtung schneidet; und
das Paar von Laufrädern einen Abschnitt beinhaltet, an dem die Laufräder einander in der Aufwärts-Abwärtsrichtung überlappen.

7. Verfahren zur Herstellung eines saugfähigen Artikels nach Anspruch 6, wobei:
das vordere äußere Element (30) und das hintere äußere Element (40) zwischen der Vorderseite und der Rückseite des saugfähigen Hauptkörpers (10) in der Aufwärts-Abwärtsrichtung durch das Paar von Laufrädern eingeklappt sind, das gegen Positionen an dem vorderen äußeren Element (30) und dem hinteren äußeren Element (40), die sich von den Positionen der Vereinigungsabschnitte unterscheiden, drücken.

8. Verfahren zur Herstellung eines saugfähigen Artikels nach Anspruch 6 oder Anspruch 7, wobei:
eine Rotationswelle von mindestens einem Laufrad aus dem Paar von Laufrädern in einem vorbestimmten Neigungswinkel zur Aufwärts-Abwärtsrichtung angeordnet ist.

9. Verfahren zur Herstellung eines saugfähigen Artikels nach einem von Anspruch 1 bis Anspruch 5, wobei:
das vordere äußere Element (30) und das hintere äußere Element (40) zwischen der Vorderseite und der Rückseite des saugfähigen Hauptkörpers (10) in der Aufwärts-Abwärtsrichtung durch Ausstoßen von Luft aus einem Paar von Gebläsedüsen eingeklappt sind, die jeweils an jeder Seite des überziehbaren saugfähigen Artikels (1) in einer Richtung, die die Förderrichtung schneidet, um die Luft gegen das vordere äußere Element (30) und das hintere äußere Element zu blasen, bereitgestellt sind.

10. Verfahren zur Herstellung eines saugfähigen Artikels nach einem von Anspruch 1 bis Anspruch 9, wobei das Verfahren zur Herstellung eines saugfähigen Artikels Folgendes beinhaltet:
einen Prozess, bei welchem ein vorderes bandförmiges Element, das durch eine Vielzahl der vorderen äußeren Elemente konfiguriert ist, die zusammenhängend entlang der seitlichen Richtung miteinander verbunden sind, und ein hinteres bandförmiges Element, das durch eine Vielzahl der hinteren äußeren Elemente konfiguriert ist, die zusammenhängend entlang der seitlichen Richtung miteinander verbunden sind, entlang der seitlichen Richtung befördert werden, und, während der Beförderung entlang der seitlichen Richtung, das vordere bandförmige Element und das hintere bandförmige Element bei jeweiligen Stirnabschnitten in der seitlichen Richtung der vorderen äußeren Elemente und der hinteren äußeren Elemente geschnitten werden; und
einen Prozess, bei welchem eine Richtung, in welcher das vordere bandförmige Element und das hintere bandförmige Element befördert werden, durch einen vorbestimmten Winkel derart umgedreht wird, dass, wenn sie geschnitten wurden, die geschnittenen vorderen bandförmigen Elemente und die geschnittenen hinteren bandförmigen Elemente entlang der Höhenrichtung befördert werden.

## Revendications

1. Procédé de fabrication d'un article absorbant (1) pour fabriquer un article absorbant (1) à enfiler ayant une direction de hauteur, une direction latérale, et une direction avant-arrière se croisant les uns et les autres, l'article absorbant (1) comprenant un corps principal absorbant (10) conçu pour absorber des excréments, un élément extérieur avant (30) agencé au niveau d'un côté d'extrémité du corps principal absorbant (10), et un élément extérieur arrière (40) agencé au niveau d'un autre côté d'extrémité du corps principal absorbant, l'élément extérieur avant (30) et l'élément extérieur arrière (40) étant joints ensemble au niveau d'une paire de parties de raccord, le procédé de fabrication d'un article absorbant (1) comprenant :
dans un état dans lequel la direction avant-arrière de l'article absorbant (1) à enfiler est alignée dans une direction haut-bas et l'article absorbant (1) à enfiler est transporté dans une direction de transport le long de la direction de hauteur,
le fait de tirer au moins une partie sur le côté avant du corps principal absorbant (10) vers un premier côté dans la direction haut-bas et de tirer au moins une partie sur le côté arrière du corps principal absorbant (10) vers l'autre côté dans la direction haut-bas ; et
le pliage de l'élément extérieur avant (30) et de l'élément extérieur arrière (40) entre le côté avant et le côté arrière du corps principal absorbant (10) dans la direction haut-bas jusqu'à ce qu'une partie d'extrémité dans la direction latérale au niveau du côté avant du corps principal absorbant (10) soit incurvée vers l'autre côté dans la direction haut-bas et qu'une partie d'extrémité dans la direction latérale au niveau du côté arrière du corps principal absorbant (10) soit incurvée vers le premier côté dans la direction haut-bas, dans lequel lors du pliage, l'élément extérieur avant (30) et l'élément extérieur arrière (40) sont pliés entre le côté avant et le côté arrière du corps principal absorbant (10) de sorte qu'une surface d'une partie repliée de l'élément extérieur avant (30) et de l'élément extérieur arrière (40) pliée entre le côté avant et le côté arrière du corps principal absorbant (10) dans la direction haut-bas au niveau d'un côté d'ouverture de taille dans la direction de hauteur est plus grande qu'une surface d'une partie repliée de l'élément extérieur avant (30) et de l'élément extérieur arrière (40) pliée entre le côté avant et le côté arrière du corps principal absorbant (10) dans la direction haut-bas au niveau d'un côté d'entrejambe dans la direction de hauteur.

2. Procédé de fabrication d'un article absorbant selon la revendication 1, dans lequel :
quand l'élément extérieur avant (30) et l'élément extérieur arrière (40) sont repliés, une partie de chevauchement dans la direction haut-bas est réalisée là où l'élément extérieur avant (30) et l'élément extérieur arrière (40) agencés au niveau d'un côté dans la direction latérale se chevauchent avec l'élément extérieur avant (30) et l'élément extérieur arrière (40) agencés au niveau d'un autre côté dans la direction latérale.

3. Procédé de fabrication d'un article absorbant selon la revendication 2, dans lequel :
au niveau de la partie de chevauchement dans la direction haut-bas là où l'élément extérieur avant (30) et l'élément extérieur arrière (40) agencés au niveau du premier côté de direction latérale se chevauchent avec l'élément extérieur avant (30) et l'élément extérieur arrière (40) agencés au niveau de l'autre côté de direction latérale,
l'élément extérieur avant (30) et l'élément extérieur arrière (40) sont pliés vers l'intérieur de sorte que l'élément extérieur avant (30) et l'élément extérieur arrière (40) agencés au niveau du premier côté de direction latérale viennent en contact avec l'élément extérieur avant (30) et l'élément extérieur arrière (40) agencés au niveau de l'autre côté de direction latérale.

4. Procédé de fabrication d'un article absorbant selon l'une quelconque des revendications 1 à 3, dans lequel :
le corps principal absorbant (10) se contracte dans la direction latérale en raison d'une région élastique qui s'étire et se contracte le long de la direction latérale ; et
une quantité de contraction dans la direction latérale du corps principal absorbant (10) au niveau du côté arrière est différente d'une quantité de contraction dans la direction latérale du corps principal absorbant (10) au niveau du côté avant.

5. Procédé de fabrication d'un article absorbant selon la revendication 4, dans lequel :
une surface d'une partie où le corps principal absorbant (10) est tiré vers le premier côté dans la direction haut-bas au niveau du côté avant est différente d'une surface d'une partie où le corps principal absorbant (10) est tiré vers l'autre côté dans la direction haut-bas au niveau du côté arrière.

6. Procédé de fabrication d'un article absorbant selon l'une quelconque des revendications 1 à 5, dans lequel :
l'élément extérieur avant (30) et l'élément extérieur arrière (40) sont repliés entre le côté avant et le côté arrière du corps principal absorbant (10) dans la direction haut-bas par une paire de roues à palettes respectivement fournies de chaque côté de l'article absorbant (1) à enfiler dans une direction croisant la direction de transport ; et
la paire de roues à palettes comprend une partie où les roues à palettes se chevauchent l'une et l'autre dans la direction haut-bas.

7. Procédé de fabrication d'un article absorbant selon la revendication 6, dans lequel :
l'élément extérieur avant (30) et l'élément extérieur arrière (40) sont repliés entre le côté avant et le côté arrière du corps principal absorbant (10) dans la direction haut-bas par la paire de roues à palettes poussant contre des positions sur l'élément extérieur avant (30) et l'élément extérieur arrière (40) différentes des positions des parties de raccord.

8. Procédé de fabrication d'un article absorbant selon la revendication 6 ou la revendication 7, dans lequel :
un arbre de rotation d'au moins une roue à palettes parmi la paire de roues à palettes est agencé à un angle d'inclinaison prédéterminé par rapport à la direction haut-bas.

9. Procédé de fabrication d'un article absorbant selon l'une quelconque des revendications 1 à 5, dans lequel :
l'élément extérieur avant (30) et l'élément extérieur arrière (40) sont repliés entre le côté avant et le côté arrière du corps principal absorbant (10) dans la direction haut-bas en éjectant de l'air à partir d'une paire de buses de soufflage respectivement fournies de chaque côté de l'article absorbant (1) à enfiler dans une direction croisant la direction de transport de manière à souffler l'air contre l'élément extérieur avant (30) et l'élément extérieur arrière.

10. Procédé de fabrication d'un article absorbant selon l'une quelconque des revendications 1 à 9, dans lequel le procédé de fabrication d'un article absorbant comprend :
un procédé dans lequel un élément avant en forme de bande conçu par une pluralité des éléments extérieurs avant reliés ensemble de manière contiguë le long de la direction latérale, et un élément arrière en forme de bande conçu par une pluralité des éléments extérieurs arrière reliés ensemble de manière contiguë le long de la direction latérale, sont transportés le long de la direction latérale, et tout en étant transportés le long de la direction latérale, l'élément avant en forme de bande et l'élément arrière en forme de bande sont coupés au niveau de parties d'extrémité respectives dans la direction latérale des éléments extérieurs avant et des éléments extérieurs arrière ; et
un procédé dans lequel une direction dans laquelle l'élément avant en forme de bande et l'élément arrière en forme de bande sont transportés est tournée d'un angle prédéterminé de sorte que, quand ils ont été coupés, les éléments avant en forme de bande coupés et les éléments arrière en forme de bande coupés sont transportés le long de la direction de hauteur.
